Europäisches Patentamt

European Patent Office

Office européen des brevets

(11) Publication number: 0 242 075
A1

# EUROPEAN PATENT APPLICATION

(21) Application number: 87302700.7

(22) Date of filing: 27.03.87

(51) Int. Cl.4: **C12N 9/44** , C12N 1/20 , C12P 19/20 , C12P 19/22 , //(C12N9/44,C12R1:465),(C12N-1/20,C12R1:465)

(30) Priority: 16.04.86 GB 8609288

(43) Date of publication of application:
21.10.87 Bulletin 87/43

(84) Designated Contracting States:
BE DE ES FR GB NL

(71) Applicant: IMPERIAL CHEMICAL INDUSTRIES PLC
Imperial Chemical House Millbank
London SW1P 3JF(GB)

(72) Inventor: Byrom, David
27 Moor Park Nunthorpe
Middlesborough Cleveland TS7 0JJ(GB)
Inventor: Collins, Stephen Hugh
76 Cropwell Road Radcliffe-on-Trent
Nottingham N12 2SG(GB)

(74) Representative: Aufflick, James Neil et al
Imperial Chemical Industries PLC Legal
Department: Patents PO Box 6 Bessemer
Road
Welwyn Garden City Herts, AL7 1HD(GB)

(54) Debranching enzyme.

(57) A debranching enzyme of the pullulanase type derived from the novel <u>Streptomyces</u> strain NCIB 12235. The enzyme exhibits optimum activity at a pH in the range 4 to 6 and at a temperature in the range 55° to 65°C. A method for the production of the enzyme and a process for the saccharification of starch or a starch hydrolysate using the enzyme and glucoamylase or beta amylase are also disclosed.

EP 0 242 075 A1

## Debranching enzyme

This invention relates to a debranching enzyme product comprising a novel debranching enzyme of the pullulanase type, to a method for the preparation of the product and to a process for the saccharification of starch to glucose and/or other sugars using the product.

In recent years an increasing amount of attention has been given to the enzymic hydrolysis of starch to produce sugar-containing syrups, particularly glucose containing syrups. Considerable research effort has been devoted to developing more efficient processes for the enzymic hydrolysis of starch.

Starch is essentially a glucose polymer with the glucose units linked into chains by alpha 1,4-glycosidic bonds, branch chains being formed by alpha-1,6-glycosidic bonds. In the process generally employed for enzymic hydrolysis of starch, the sequential steps of liquefaction and saccharification are carried out using the enzymes alphaamylase and glucoamylase respectively. Alpha-amylase breaks 1,4-glycosidic bonds, producing oligosaccharides, whilst glucoamylase breaks 1,4-glycosidic bonds in such oligosaccharides and produces individual glucose molecules. However alpha-amylase does not break alpha-1,6-glycosidic bonds and these are broken only slowly by glucoamylase. β-amylase is used instead of glucoamylase when maltose is to be produced. This leads to a product which contains, in addition to glucose molecules, small groups of glucose units containing branched chains formed by alpha-1,6-glycosidic bonds. If the time of incubation or the concentration of glucoamylase is increased in order to achieve complete hydrolysis of the 1,6-links this unfortunately results in the formation of condensation products such as isomaltose so that it is not possible to increase the yield of glucose in this way. The production of condensation products lowers the efficiency of the process in converting starch to glucose. Since the glucose producer wishes to attain rapid digestion and the greatest possible yield of glucose the production of condensation products is a serious disadvantage.

Pullulanase is an enzyme capable of breaking the alpha-1,6-glycosidic bond in pullulan. It has been proposed to convert starch to glucose by treatment with alpha-amylase followed by treatment with glucoamylase in combination with pullulanase. This should lead to the more efficient conversion of the oligosaccharides produced by the alphaamylase into glucose molecules since the pullulanase will more effectively break the alpha-1,6-glycosidic bonds in these oligosaccharides. US Patent No. 3897305 proposes a process in which use of glucoamylase is combined with use of pullulanase derived from Aerobacter aerogenes (Klebsiella pneumoniae). A serious disadvantage of this process however is that the optimum operating conditions of temperature and pH for the particular pullulanase enzyme proposed are less than 60°C and pH 7 respectively whereas the optimum pH for glucoamylase is in the range pH 4 to pH 5 and it is preferred to operate the saccharification process at a temperature of 60°C (or at slightly higher temperatures) to avoid bacterial contamination. In UK specification GB 2097405A there is proposed a process for conversion of starch into glucose and/or other simple sugars such as maltose which employs a pullulanase derived from Bacillus acidopullulyticus. It is claimed that the optimum operating conditions of temperature and pH for the pullulanase employed in this process are more favourable.

According to the present invention we provide a debranching enzyme product which comprises a novel debranching enzyme of the pullulanase type which (a) is derived from strain NCIB 12235 or which exhibits enzyme chemical properties essentially the same as those of the debranching enzyme derived from strain NCIB 12235, (b) exhibits optimum activity at a pH in the range 4 to 6 and at a temperature in the range 55° to 65°, and (c) is obtainable from a culture produced by cultivating in a suitable nutrient medium a bacterial strain belonging to the order Actinomycetales.

Further according to the invention we provide a method for the preparation of a debranching enzyme product comprising a debranching enzyme of the pullulanase type, which enzyme is derived from strain NCIB 12235 or which exhibits enzyme chemical properties essentially the same as those of the debranching enzyme derived from strain NCIB 12235 and exhibits optimum activity at a pH in the range 4 to 6 and at a temperature in the range 55° to 65°C, wherein a bacterial strain of the order Actinomycetales is cultivated in a suitable nutrient medium containing sources of carbon and inorganic nutrients under conditions suitable for the production of the debranching enzyme and the debranching enzyme product is recovered.

Further according to the present invention we provide a process for the saccharification of starch or a starch hydrolysate to produce a sugar-containing syrup which comprises a step in which the starch or starch hydrolysate is treated with glucoamylase or beta amylase wherein before or during this step the starch or starch hydrolysate is treated with a debranching enzyme product comprising a debranching enzyme of the pullulanase type which exhibits optimum activity at a pH in the range 4 to 6 and at a temperature in the range 55° to 65°, which product is produced by cultivating a bacterial strain belonging to the genus Streptomyces in a suitable nutrient medium containing sources of carbon and inorganic nutrients under conditions suitable for the production of the debranching enzyme.

Further according to the present invention we provide a biologically pure culture of the bacterial strain NCIB 12235 or of a variant or mutant derived from this strain.

The saccharification process of the invention may be carried out using a debranching enzyme product derived from any suitable bacterial strain of the genus Streptomyces. An especially suitable strain is the novel Streptomycete which we have isolated from leaf litter and a culture of which has been deposited on 27 March 1986 at the National Collection of Industrial and Marine Bacteria (NCIMB), Torry Research Station Aberdeen, Scotland, UK where it has been assigned the accession number NCIB 12235. This novel strain has been grown on Pullulan at pH 5 and 60° and shows pullulanase activity.

The characteristics of Streptomyces strain NCIB 12235 are as follows:-]

Morphological properties

Colonies cream, sparse aerial mycelium, grey/white aerial spore mass.

Degradation tests

Positive: Gelatin, hypoxanthine, Tween 20, 40 and 60, tyrosine
Negative : Elastin, xanthime

Utilisation as sole carbon source

10 g/l⁻¹

Positive: L-arabinose, D-galactose, D-gluconic acid, D-glucosamine HCl, D-glucose, D-lactose, maltose, D-melibiose, glycerol, glycogen, D-raffinose, D-trehalose
Negative: D-amygdaline, meso-erythritol, meso-inositol, inulin, D-melezitose, α-methyl-D-glucoside, L-rhamnose, D-ribose, D-sorbitol

1 g/l⁻¹

Positive: L-alanine, L-aspartic acid, D-glutamic acid, L-iso-leucine, L-phenylalanine, sodium succinate
Negative: Acetamide, Adipic acid, propan-1-ol, sodium oxalate, sodium propionate, sodium pyruvate, p-hydroxybenzoic acid

Utilisation as sole carbon and nitrogen source

1 g/l⁻¹

Positive: L-glutamic acid, L-phenylalanine
Negative: Acetamide, sodium hippurate

Tolerance to antibiotics (μg/ml⁻¹)

Resistance to:

Gentamycin sulphate (32), lincomycin hydrochloride (16), neomycin sulphate (32), oleandomycin phosphate (4), penicillin G (sodium salt, 2), streptomycin sulphate (4), tobramycin sulphate (32)

Sensitive <u>to</u>:

Cephaloridine hydrochloride (2), chlortetracycline hydrochloride (2), domeclocycline (2), oleandomycin phosphate (32), penicillin-G (sodium salt, 16), rifampicin (4), vancomycin hydrochloride (2)

Growth <u>at</u>:

10, 15 and 30°C and at pH 4.5 to 6

<u>Growth in presence of</u> ($\mu$g/ml$^{-1}$)

Copper sulphate (10), crystal violet (1), ferrous sulphate (50), lead acetate (100), manganese sulphate (100), potassium tellurite (50).

A suitable growth medium for strain NCIB 12235 has the following composition (concentrations are expressed in g/l except where stated):

$K_2SO_4$ 0.1

$K_2HPO_4$ 0.3

$FeSO_4$ 0.05

"Difco" casitone 2.0

"Oxoid yeast extract 1.0

1.1 M phosphoric acid 11.2 mls.

$MgSO_4.7H_2O$ 0.8

Trace metals solution 18 mls.

Maltose 15

The pH was controlled to 4.5 by addition of ammonium hydroxide. Agar in an amount of 15 g/l was added to give a solid medium.

In addition to the above medium we believe that strain NCIB 12235 can be grown on most salts solutions when added to glucose, maltose or starch together with casitone and yeast extract.

The saccharification process of the invention is preferably used to produce glucose-containing syrups, i.e. syrups containing glucose as the main sugar component. It may however also be used to produce syrups containing other sugars in particular maltose as the main sugar component. Preferably before treatment with the novel debranching enzyme and glucoamylase or $\beta$-amylase (if maltose is to be the main product) the starch is subjected to a liquefaction step to produce a starch hydrolysate. The starch or starch hydrolysate may be treated with the novel debranching enzyme before it is treated with glucoamylase or $\beta$-amylase but preferably it is treated with the debranching enzyme and glucoamylase or $\beta$-amylase at the same time. In the preferred mode of operating the process of the invention, starch is treated first with alpha-amylase and the resulting hydrolysate is then treated with glucoamylase or $\beta$-amylase and the novel debranching enzyme of the invention together. Preferably the glucoamylase or $\beta$-amylase and the novel debranching enzyme are used to treat a starch hydrolysate having a dry solids content of at least 30% by weight. Preferably saccharification takes place at a temperature in the range 55° to 65° and at a pH in the range 4 to 6. The most preferred temperature range is 55° to 60° in which case enzymes having optimum activity at temperatures within this range are used.

Preferably the alpha-amylase enzymes employed are derived from strains of <u>Bacillus</u>. Preferred glucoamylase enzymes are derived from fungal strains whilst $\beta$-amylase enzymes may be derived from malt or from bacterial sources.

The invention is illustrated by the following Examples:-

EXAMPLE 1

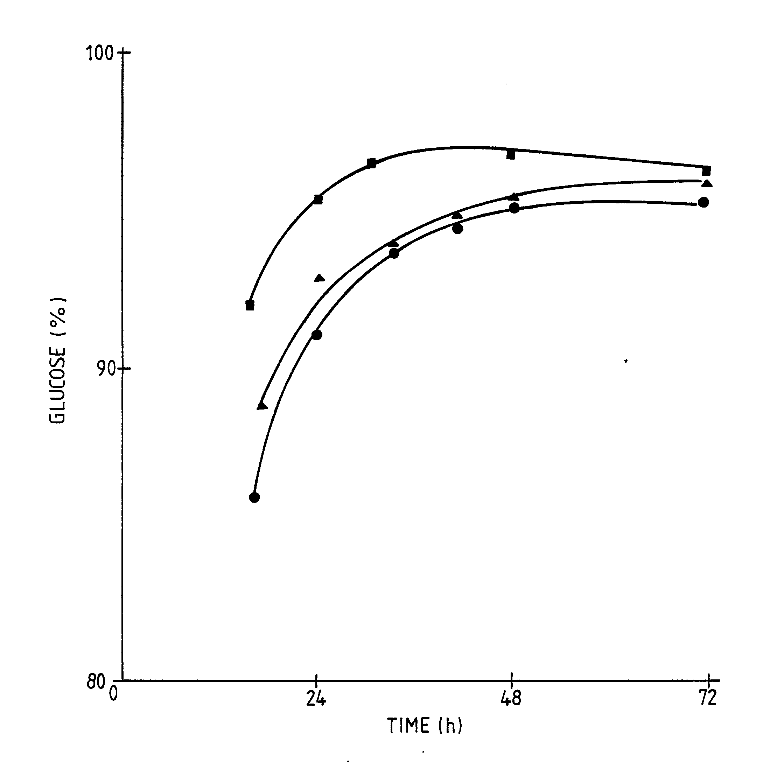

A 30% $^w$/w solution of a commercially available liquified starch ("Maldex 15") in 100 mM acetate buffer plus 5 mM calcium chloride was incubated with glucoamylase (0.9 $\mu$l "Optidex 2004L" per g starch) and pullulanase of the invention (1 unit/g starch) at 60° and pH 4.2.) Samples were taken at intervals up to 72 hr and glucose concentrations (as percentages of total sugars) were determined by high pressure liquid chromatography (HPLC). The results are shown in the Figure in which these determinations are denoted by the squares on the graph. The Figure also shows the results obtained with comparative incubations carried out using glucoamylase alone (determinations denoted by circles) and with the pullulanase of the invention replaced by a pullulanase having the same activity derived from Bacillus acidopullulyticus (determinations denoted by triangles).

The Figure shows that use of the pullulanase of the invention produces an increase in maximum glucose concentration of approximately 1.5% with this maximum being reached at approximately 24 hr rather than the 48 to 72 hr as is the case without pullulanase or with the pullulanase from Bacillus accidopullulyticus . Bacillus acidopullulyticus pullulanase is ineffective under the conditions of this example, producing yields of glucose only marginally better than those produced without any pullulanase.

## EXAMPLE 2

A series of incubations similar to that described in Example 1 was performed, the only difference being that the pH was raised to 4.5. The results obtained without pullulanase and with the pullulanase of the invention were as described in Example 1. However at the higher pH the pullulanase from Bacillus acidopullulyticus produced results comparable with the results obtained using the pullulanase of the invention.

## EXAMPLE 3

A series of incubations similar to that described in Example 1 was performed, the only difference being that the pH was lowered to pH 4.0. At this lower pH the results obtained using pullulanase from Bacillus acidopullulyticus were the same as those obtained without pullulanase. The pullulanase of the invention produced an increase in maximum glucose yield of approximately 1.1%, the maximum being reached between 36 and 48 hr.

## EXAMPLE 4

A 30% ʷ/w solution of a commercially available liquified starch ("Maldex 15") in 100 mM acetate buffer plus 5 mM calcium chloride was incubated at pH 5.0 and 60°C with barley β-amylase ("Spezyme BBA 1500"), present in the ratio of 0.75 μl/g starch, and 1 unit/g starch of the pullulanase of the invention. α-amylase ("Optitherm 2420") was added in the ratio 0.5 μl/g substrate 6 hr after the start of the incubation. Samples were withdrawn at intervals up to 72 hr and their carbohydrate composition was determined by HPLC. Comparative incubations were performed without pullulanase and with a pullulanase derived from Bacillus acidopullulylicus. In all cases no further change in the carbohydrate composition was noted after 24 hr. The results are set out in the Table which gives the carbohydrate composition at the 24 hr point. The results show that both pullulanases are similarly effective in promoting the digestion of liquified starch to produce maltose.

### Table

| Carbohydrate | Percentage present in product of digestion after 24 hours. | | |
|---|---|---|---|
| | control (No pullulanase) | B. Acido- Pullulyticus Pullulanase | Pullulanase of the Invention |
| Glucose | 2 | 2 | 1 |
| Maltose | 49 | 58 | 60 |
| Maltotriose | 20 | 28 | 26 |
| Oligosaccharides | 29 | 12 | 12 |

**Claims**

1. A debranching enzyme product which comprises a debranching enzyme of the pullulanase type which (a) is derived from strain NCIB 12235 or which exhibits enzyme chemical properties essentially the same as those of the debranching enzyme derived from strain NCIB 12235, (b) exhibits optimum activity at a pH in the range 4 to 6 and at a temperature in the range 55° to 65°, and (c) is obtainable from a culture produced by cultivating in a suitable nutrient medium a bacterial strain belonging to the order Actinomycetales.

2. A biologically pure culture of the bacterial strain NCIB 12235 or of a variant or mutant derived from this strain.

3. A method for the preparation of a debranching enzyme product comprising a debranching enzyme of the pullulanase type, which enzyme is derived from strain NCIB 12235 or which exhibits enzyme chemical properties essentially the same as those of the debranching enzyme derived from strain NCIB 12235 and exhibits optimum activity at a pH in the range 4 to 6 and at a temperature in the range 55° to 65°C, wherein a bacterial strain of the order Actinomycetales is cultivated in a suitable nutrient medium containing sources of carbon and inorganic nutrients under conditions suitable for the production of the debranching enzyme and the debranching enzyme product is recovered.

4. A process for the saccharification of starch or a starch hydrolysate to produce a sugar-containing syrup which comprises a step in which the starch or starch hydrolysate is treated with glucoamylase or beta amylase wherein before or during this step the starch or starch hydrolysate is treated with a debranching enzyme product comprising a debranching enzyme of the pullulanase type which exhibits optimum activity at a pH in the range 4 to 6 and at a temperature in the range 55° to 65°, which product is produced by cultivating a bacterial strain belonging to the genus Streptomyces in a suitable nutrient medium containing sources of carbon and inorganic nutrients under conditions suitable for the production of the debranching enzyme.

5. A process according to claim 4 wherein glucoamylase is used and the sugar-containing syrup produced contains glucose as its main sugar component.

6. A process according to claim 4 wherein Beta amylase is used and the sugar-containing syrup produced contains maltose as its main sugar component.

7. A process according to any one of claims 4 to 6 wherein starch is treated with alpha amylase to produce a starch hydrolysate which is thereafter treated with the debranching enzyme and glucoamylase or beta amylase together.

8. A process according to claim 7 wherein the starch hydrolysate which is treated with the debranching enzyme and glucoamylase or beta amylase has a dry solids content of at least 30% by weight.

9. A process according to any one of claims 4 to 8 wherein treatment with the debranching enzyme takes place at a temperature in the range 55 to 60°C.

10. A process according to claim 7 or claim 8 wherein the alpha amylase is derived from a strain of the genus Bacillus, the glucoamylase is derived from a fungal strain and the beta amylase is derived from malt or from bacterial sources.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 86, no. 17, 25th April 1977, pages 386-387, abstract no. 119268j, Columbus, Ohio, US; & JP-A-76 125 791 (MEITO SANGYO CO. LTD) 02-11-1976 * Whole abstract * | 1,3 | C 12 N 9/44<br>C 12 N 1/20<br>C 12 P 19/20<br>C 12 P 19/22 //<br>(C 12 N 9/44<br>C 12 R 1:465)<br>(C 12 N 1/20<br>C 12 R 1:465) |
| X | --- CHEMICAL ABSTRACTS, vol. 84, no. 5, 2nd February 1976, page 318, abstract no. 29206s, Columbus, Ohio, US; & JP-A-75 111 278 (NIKKEN CHEMICAL CO., LTD) 01-09-1975 * Whole abstract * | 1,3 | |
| X | --- FR-A-2 069 320 (HAYASHIBARA CO.) * Claims * | 1,3 | |
| X | --- DE-A-2 031 269 (HAYASHIBARA CO.) * Claim 2; pages 4-5 * | 1,7 | **TECHNICAL FIELDS SEARCHED (Int. Cl.4)**<br><br>C 12 N<br>C 12 P<br>C 12 R |
| D,X | --- EP-A-0 063 909 (NOVO INDUSTRI A/S) * Claims 12-14 * | 4-9 | |
| X | --- GB-A-1 144 950 (CORN PRODUCTS CO.) * Claims 1,3,5-10 * | 4,6-10 | |
| | --- -/- | | |

The present search report has been drawn up for all claims

| Place of search | Date of completion of the search | Examiner |
|---|---|---|
| THE HAGUE | 23-07-1987 | ALVAREZ Y ALVAREZ C. |

## DOCUMENTS CONSIDERED TO BE RELEVANT

Page  2

| Category | Citation of document with indication, where appropriate, of relevant passages | Relevant to claim | CLASSIFICATION OF THE APPLICATION (Int. Cl.4) |
|---|---|---|---|
| P,X | EP-A-0 188 049  (AGENCY OF INDUSTRIAL SCIENCE & TECHNOLOGY MINISTRY OF INTERNATIONAL TRADE & INDUSTRY) <br> * Claims 1-2,7-8; examples 5,9 * | 4-7,9-10 | |
| A | CHEMICAL ABSTRCACTS, vol. 62, no. 3, 1st February 1965, columns 2970h - 2971b, Columbus, Ohio, US; T. OHTSUKI et al.: "Degradation of starch by some actinomycetales", & NIPPON KIN GAKKAI KAIHO 3(1), 1-6(1962) <br> * Whole abstract * | 10 | |
| A | PATENT ABSTRACTS OF JAPAN, vol. 4, no. 12 (C-71), 29th January 1980, page 107 C 71; & JP-A-54 147 994 (KOGYO GIJUTSUIN) 19-11-1979 <br> * Whole abstract * | 10 | TECHNICAL FIELDS SEARCHED (Int. Cl.4) |
| A | CHEMICAL ABSTRACTS, vol. 87, no. 1, 4th July 1977, page 334, abstract no. 3999q, Columbus, Ohio, US; M. DREIMANE et al.: "Production of an industrial-grade glucoamylase enzyme preparation and its biological value", & MIKROBIOL. PREP. 1976, 142-56 <br> * Whole abstract * | 10 | |

--- -/-

The present search report has been drawn up for all claims

| Place of search | Date of completion of the search | Examiner |
|---|---|---|
| THE HAGUE | 23-07-1987 | ALVAREZ Y ALVAREZ C. |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | GB-A-1 332 770 (AJINOMOTO CO. INC.)<br>* Claim 2 * | 10 | |

-----

TECHNICAL FIELDS SEARCHED (Int. Cl.4)

The present search report has been drawn up for all claims

| Place of search | Date of completion of the search | Examiner |
|---|---|---|
| THE HAGUE | 23-07-1987 | ALVAREZ Y ALVAREZ C. |